(19) Europäisches Patentamt
European Patent Office
Office européen des brevets

(11)  EP 0 691 320 B1

(12)  **EUROPÄISCHE PATENTSCHRIFT**

(45) Veröffentlichungstag und Bekanntmachung des Hinweises auf die Patenterteilung:
**05.03.1997 Patentblatt 1997/10**

(51) Int. Cl.$^6$: **C07C 13/20**, C07C 5/11

(21) Anmeldenummer: **95110139.3**

(22) Anmeldetag: **29.06.1995**

(54) **Verfahren zur kontinuierlichen Herstellung von Cyclohexen durch partielle Hydrierung von Benzol**

Process for the continuous preparation of cyclohexene by partial hydrogénation of benzene

Procédé pour la préparation en continu de cyclohexène par hydrogénation partielle de benzène

(84) Benannte Vertragsstaaten:
**BE CH DE ES FR GB IT LI NL**

(30) Priorität: **08.07.1994 DE 4423725**

(43) Veröffentlichungstag der Anmeldung:
**10.01.1996 Patentblatt 1996/02**

(73) Patentinhaber: **BASF AKTIENGESELLSCHAFT**
**67056 Ludwigshafen (DE)**

(72) Erfinder:
- **Gaube, Johann, Prof. Dr.**
  **D-64380 Rossdorf (DE)**
- **Doebert, Frank**
  **D-51065 Köln (DE)**

(56) Entgegenhaltungen:
**EP-A- 0 055 495**

**Beschreibung**

Die vorliegende Erfindung betrifft ein verbessertes Verfahren zur kontinuierlichen Herstellung von Cyclohexen durch partielle Hydrierung von Benzol mit Wasserstoff in Gegenwart von Wasser und Rutheniumkatalysatoren bei erhöhter Temperatur und erhöhtem Druck.

Die US 4,678,861 beschreibt die partielle Hydrierung von Benzol zu Cyclohexen diskontinuierlich in einer Suspension, wobei man die Reaktion in einem zweiphasigen, flüssigen Gemisch durchführt. Nachteilig an dieser Verfahrensweise ist die Abtrennung des Katalysators aus der organischen Phase und gegebenenfalls der Austrag von Salzen.

Die EP-A 552 809 beschreibt die partielle Hydrierung von Benzol zu Cyclohexen in einem System bestehend aus einer wäßrigen Phase, enthaltend den darin suspendierten Katalysator, einer öligen Phase, enthaltend den zu hydrierenden Kohlenwasserstoff, und eine gasförmige Phase aus Wasserstoff. Nachteilig an dieser Verfahrensweise ist u.a. das Unterbrechen der Reaktion zur Trennung der organischen von der wäßrigen Phase.

Die EP-B 55 495 beschreibt die partielle Hydrierung von Benzol zu Cyclohexen in der Gasphase, wobei eine Ausbeute an Cyclohexen von maximal nur 8,4 % erreicht wird.

Aufgabe der vorliegenden Erfindung war es daher, ein verbessertes Verfahren zur kontinuierlichen Herstellung von Cyclohexen durch partielle Hydrierung von Benzol mit Wasserstoff in Gegenwart von Wasser und Rutheniumkatalysatoren bei erhöhter Temperatur und erhöhtem Druck bereitzustellen, das die oben genannten Nachteile nicht aufweist.

Demgemäß wurde ein Verfahren zur kontinuierlichen Herstellung von Cyclohexen durch partielle Hydrierung von Benzol mit Wasserstoff in Gegenwart von Wasser und Rutheniumkatalysatoren bei erhöhter Temperatur und erhöhtem Druck gefunden, indem man Benzol gasförmig zuführt und gebildetes Cyclohexen gasförmig austrägt, wobei der Katalysator in einer flüssigen, wäßrigen Phase gelöst oder suspendiert vorliegt.

Erfindungsgemäß leitet man Benzol und Wasserstoff, gemeinsam oder getrennt, gasförmig zu und trägt das gebildete Cyclohexen gasförmig aus. Vorteilhaft führt man Benzol mit einem Inertgas als Träger in den Reaktionsraum ein, wobei man bevorzugt das Inertgas wie Stickstoff, Helium oder Argon durch eine Benzol enthaltende Sättigungsvorrichtung leitet, wobei Benzol gasförmig aufgenommen ("beladen") wird. Üblicherweise erfolgt die Beladung des Inertgasstromes bei einer Temperatur im Bereich von 20 bis 250°C, vorzugsweise von 50 bis 170°C, wobei man vorzugsweise hierzu das Inertgas vor der Sättigungseinrichtung erhitzt. Zweckmäßig wählt man den Druck nach der Sättigungsvorrichtung so, daß er dem Gesamtdruck im Reaktionsraum entspricht.

Bei Verwendung eines Inertgases als Trägergas für Benzol wählt man die Benzolkonzentration üblicherweise im Bereich von 0,1 bis 99,9 Vol.-%, bevorzugt von 0,5 bis 95 Vol.-%.

Das gasförmige Benzol, mit oder ohne Trägergas, führt man erfindungsgemäß dem Hydrierkatalysator zu, der sich, gelöst oder suspendiert, in einer flüssigen, wäßrigen Phase befindet. Den Benzolpartialdruck im Reaktionsraum wählt man im allgemeinen im Bereich von 10 kPa bis 1 MPa, vorzugsweise von 150 kPa bis 0,5 MPa.

Nach den bisherigen Beobachtungen kann man das gasförmige Benzol auf verschiedene Art und Weise in den Reaktionsraum zuführen, beispielsweise mit oder ohne eine Düse, über ein einfaches Einleitrohr, in die flüssige, wäßrige Phase oder oberhalb der Flüssigkeitsoberfläche.

Den Wasserstoffpartialdruck im Reaktionsraum wählt man in der Regel im Bereich von 50 kPa bis 5 MPa, vorzugsweise von 0,5 MPa bis 4 MPa.

Vorteilhaft führt man die Umsetzung bei Temperaturen im Bereich von 20 bis 300, vorzugsweise von 100 bis 200°C durch.

Die Druck- und Temperaturbedingungen stimmt man zweckmäßig so aufeinander ab, daß die Einhaltung einer flüssigen, wäßrigen Phase gewährleistet ist, und daß die Menge an zugeführtem Benzol der Menge des organischen Austrags, bestehend im wesentlichen aus Cyclohexen, Cyclohexan und unumgesetztem Benzol entspricht. In einer bevorzugten Ausführungsform steuert man den Druck im Reaktionsraum mit einem Inertgas wie Stickstoff, Argon oder Helium, bevorzugt Stickstoff, wobei man den Gesamtdruck im Reaktionsraum im Bereich von 0,1 bis 20, vorzugsweise von 1 bis 10 MPa, wählt.

Zweckmäßig bewegt man die flüssige, wäßrige Phase, vorzugsweise durch Rühren, wobei man Rührgeschwindigkeiten in einem Bereich von 300 bis 1500, vorzugsweise von 750 bis 1500 Umdrehungen pro Minute wählt.

Als Katalysatoren kann man nach bisherigen Beobachtungen alle bekannten Ruthenium-haltigen Homogen- oder Suspensionskatalysatoren (Träger- oder Fäll-Katalysatoren) einsetzen. Solche Katalysatoren sind beispielsweise beschrieben in US 4,678,861, EP-A 220,525, WO 93/16971, WO 93/16972, EP-A 554,765, wobei Katalysatoren hergestellt gemäß Beispiel 1 aus der US 4,678,861 (Ruthenium auf einem Lanthanoxid-Träger) und Beispiel 1 aus der EP-A 220,525 (Ruthenium/Zink-Fällkatalysatoren) sowie gemäß Beispiel 1 aus der DE-A 4,203,220 (Ruthenium/Nickel-Fällkatalysatoren) besonders bevorzugt sind.

Vorteilhaft verwendet man einen Katalysator, der, bezogen auf eingesetztes Benzol, von 0,01 bis 100, vorzugsweise von 0,1 bis 80 Gew.-% Ruthenium enthält.

Die Umsetzung wird erfindungsgemäß in Gegenwart von Wasser durchgeführt. Hierbei wählt man das Gewichtsverhältnis von Wasser zu Katalysator vorzugsweise im Bereich von 5:1 bis 1000:1, besonders bevorzugt von 50:1 bis 500:1.

2

Im allgemeinen trägt man in dem Maße wie Wasser gasförmig ausgetragen wird, Wasser in den Reaktionsraum ein, wobei man das Wasser flüssig über Pumpen oder gasförmig, beispielsweise über eine Sättigungsvorrichtung, eintragen kann.

Zweckmäßig führt man Benzol und gewünschtenfalls Inertgas in solchen Mengen zu, daß der Katalysator stets vollständig, in der wäßrigen Phase vorliegt.

Je nach Katalysatorsystem kann man die Reaktion sowohl im alkalischen, neutralen als auch im sauren Bereich durchführen.

Bei einer Durchführung der Reaktion im alkalischen Milieu setzt man der wäßrigen Phase in der Regel Hydroxide der Alkalimetalle oder Erdalkalimetalle, besonders bevorzugt Natriumhydroxid und Kaliumhydroxid, im Bereich von 0,01 bis 10, vorzugsweise von 0,1 bis 5 mol/l zu.

Bei einer Durchführung der Reaktion im sauren Milieu setzt man der wäßrigen Phase in der Regel saure Salze von Übergangsmetallen oder anorganische oder organische Säuren im Bereich von 0,001 bis 10, vorzugsweise von 0,1 bis 5 mol/l zu.

Vorteilhaft enthält die wäßrige Phase ein oder mehrere gelöste Kationen von Übergangsmetallen der 2. bis 8. Gruppe des periodischen Systems wie Chrom, Mangan, Eisen, Kobalt, Kupfer, Zink sowie Ammonium in Form ihrer Chloride, Nitrate, Acetate, Phosphate oder Sulfate. Die Menge an Metallsalz, bezogen auf die wäßrige Phase, beträgt vorteilhaft von 0,01 Gew.-% bis zur Sättigungskonzentration.

Des weiteren kann es vorteilhaft sein, dem Reaktionsgemisch mindestens eines der Metalloxide, ausgewählt aus der Gruppe, bestehend aus Aluminiumoxid, Siliciumdioxid, Zirkoniumdioxid, Titandioxid, Hafniumdioxid, Chromtrioxid und Zinkoxid, zuzusetzen. Vorzugsweise beträgt die Menge an zugesetztem Metalloxid von 0,001 bis 1 Gew.-%, bezogen auf die eingesetzte Wassermenge.

Das ausgetragene Gasgemisch, enthaltend im wesentlichen Cyclohexen, Cyclohexan, unumgesetztes Benzol, Wasserstoff sowie gegebenenfalls Inertgas, wird in der Regel destillativ aufgearbeitet, indem man z.B. die organische Phase durch Kondensation vom übrigen Gemisch abtrennt, und dann daraus durch Extraktivdestillation Cyclohexen gewinnt.

In einer bevorzugten Ausführungsform verwendet man einen üblichen Rührkessel zur Durchführung der Reaktion. Durch Aneinanderreihung mehrerer Rührkessel kann ein Strömungsrohrverhalten bewirkt werden. Nach bisherigen Beobachtungen kann man auch andere Reaktortypen wie Blasensäulenreaktoren einsetzen.

Das nach dem erfindungsgemäßen Verfahren erhältliche Cyclohexen eignet sich zur Herstellung von Cyclohexanol, einem wichtigen Ausgangsstoff für die Herstellung von Faservorprodukten.

Die Vorteile des vorliegenden Verfahrens liegen darin, daß der Katalysator und vorliegende Salze nicht ausgetragen werden können, die Salzkonzentration konstant gehalten werden kann und höhere Cyclohexenselektivitäten als bisher erreicht werden.

Beispiele

Beispiel 1

In einem Rührkesselreaktor (650 ml Volumen, mit Teflon vollständig ausgekleidet), wurden 5 g eines Ruthenium-Lanthanoxidkatalysators (hergestellt gemäß Beispiel 1 aus der US 4,678,861) zusammen mit 1 g Zinkchlorid und 6,24 g Natriumhydroxid mit 250 ml destilliertem Wasser überschichtet. Nach Verschließen des Reaktors wurde ein Druck im Reaktor mit Stickstoff auf 5,0 MPa eingestellt. Danach wurde der Reaktor unter kräftigem Rühren (500 U/min) 4 h mit Stickstoff (300 Nml/min; Nml=ml, bezogen auf Normbedingungen (T= 273K, p= 101,325 kPa)) gespült und auf eine Temperatur von 130°C aufgeheizt. Danach wurde der Katalysator mit einem Wasserstoff/Stickstoff-Gemisch (zugeführte Menge 50 Nml/min Wasserstoff und 300 Nml/min Stickstoff) unter kräftigem Rühren zwölf Stunden lang aktiviert. Anschließend wurde der Stickstoffstrom durch einen mit Benzol befüllten Sättiger geleitet, wobei die Beladung des Stickstoffstromes bei einer Temperatur im Bereich von 65°C erfolgte. Dann wurde der mit Benzol beladene Stickstoffstrom (260 ml/min) mit dem Wasserstoffstrom (90 ml/min) vereinigt und dem Reaktor zugeführt.

Vor der ersten Probe (Beispiel 1a) wurde die Reaktion 6 h betrieben. Danach wurden die Benzol- und Wasserstoffpartialdrücke gemäß Tabelle 1 (Beispiele 1b und 1c) variiert, wobei die Reaktionszeiten jeweils 3 h betrugen. Eine Desaktivierung des Katalysators war über einen Zeitraum von 10 Tagen nicht zu beobachten.

Die Zusammensetzung des gasförmigen Reaktionsaustrages wurde gaschromatographisch analysiert.

Die Ergebnisse sind in Tabelle 1 zusammengefaßt.

Tabelle 1

| Bsp. 1 | Reaktionszeit [h] (insgesamt) | p(H$_2$) [kPa] | p(Benzol) [kPa] | Umsatz [%] | Selektivität [%] | Ausbeute [%] |
|--------|-------------------------------|----------------|------------------|------------|-------------------|---------------|
| a | 6 | 1523 | 62 | 14,5 | 80,4 | 11,6 |
| b | 9 | 1202 | 44 | 29,2 | 68,7 | 20,1 |
| c | 12 | 1189 | 37 | 40,5 | 57,5 | 23,3 |

Als Vergleichswerte wurde die Beispiele 42 - 44 aus der US 4,678,861 herangezogen. Die in Beispiel 1 angegeben Werte sind differentielle Selektivitäten. Um diese Werte mit den entsprechenden Werten aus der US 4,678,861 vergleichen zu können, wurden gemäß der Gleichung

$$S_{int} = \frac{1}{U_{end}} \int_{O}^{U} s_{diff}(U)dU$$

wobei $S_{int}$ = integrale Selektivität, $S_{diff}$ = differentielle Selektivität und $U_{end}$ der Umsatz am Ende der Reaktion bedeuten, die integralen Selektivitätswerte von Beispiel 1 ermittelt.

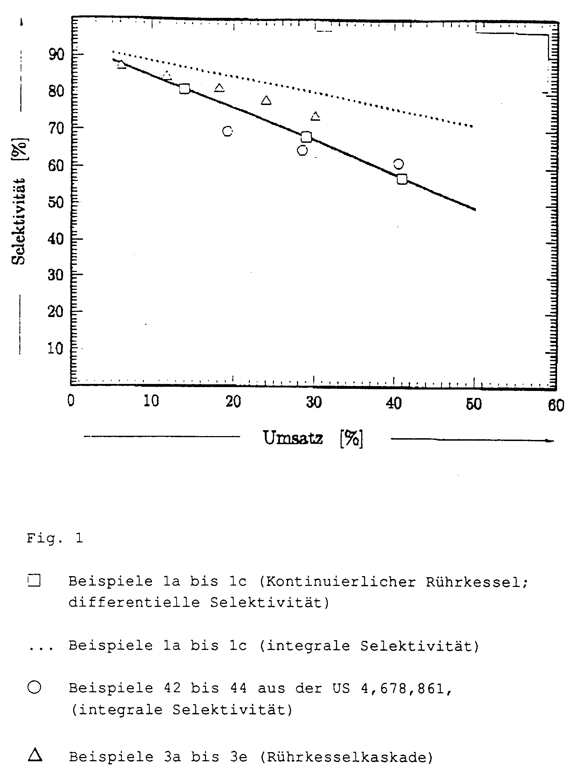

Fig. 1 zeigt die Versuchsergebnisse, wobei

O die Ergebnisse aus den Beispielen 42 bis 44 von US 4,678,861,

☐ die Versuchsergebnisse aus den Beispielen 1a bis 1c und ... die zu ☐ errechneten integralen Selektivitäten wiedergeben.

Beispiel 2

Beispiel 1 wurde mit folgenden Änderungen unter ansonsten gleichen Bedingungen wiederholt: 10,0 g Katalysator, 2,0 g Zinkchlorid und 6,32 g Natriumhydroxid.
Die Ergebnisse sind in Tabelle 2 zusammengefaßt.

Tabelle 2

| Bsp. 2 | Reaktionszeit [h] | p(H$_2$) [kPa] | p(Benzol) [kPa] | Umsatz [%] | Selektivität [%] | Ausbeute [%] |
|--------|-------------------|----------------|------------------|------------|-------------------|---------------|
| a | 12 | 764 | 86 | 14,9 | 81,9 | 12,2 |
| b | 18 | 757 | 77 | 33,3 | 67,0 | 22,3 |
| c | 24 | 743 | 57 | 40,5 | 60,8 | 24,6 |
| d | 30 | 732 | 51 | 41,1 | 61,3 | 25,2 |
| e | 36 | 786 | 35 | 44,3 | 59,1 | 26,2 |

Beispiel 3 (Rührkesselkaskade)

Zur Simulation einer Rührkesselkaskade wurde im Rührkesselreaktor aus Beispiel 1 folgendes Katalysatorsystem vorgelegt:
5,0 g Ruthenium-Lanthanoxidkatalysator (wie in Beispiel 1) 1,2 g ZnCl$_2$, 6,34 g NaOH und 250 ml destilliertes Wasser. Die Aktivierung des Katalysators wurde entsprechend den vorhergehenden Beispielen durchgeführt. Danach wurde ein Stickstoffstrom (350 Nml/min), der bei 105°C mit Benzol beladen wurde, zusammen mit Wasserstoff (75 Nml/min) dem Reaktor zugeführt. Die Zusammensetzung der Kohlenwasserstoffe im Produktstrom wurde mit einem Gaschromatographen analysiert. Anschließend wurde dem Reaktor zur Simulation des zweiten Kaskadenreaktors ein Kohlenwasserstoffgemisch zugeführt, das die Zusammensetzung des Produktgasstromes aus der ersten Umsetzung besaß.

Analog zu dieser Vorgehensweise wurden drei weitere Umsetzungen durchgeführt, so daß eine Rührkesselkaskade, bestehend aus fünf Rührkesseln, simuliert wurde.

Die Reaktionszeiten betrugen jeweils 6 h.

Die Ergebnisse sind in Tabelle 3 zusammengefaßt und in Fig. 1 bildlich (△) dargestellt.

Tabelle 3

| Reaktor Nr. | $P(H_2)$ [kPa] | $P(C_6H_6)$ [kPa] | $P(C_6H_{10})$ [kPa] | $P(C_6H_{12})$ [kPa] | Umsatz [%] | Selektivität [%] | Ausbeute [%] |
|---|---|---|---|---|---|---|---|
| 1 | 786 | 158 | 9,0 | 1,3 | 6,1 | 87,4 | 5,3 |
| 2 | 785 | 147 | 16,6 | 3,0 | 11,8 | 84,7 | 10,0 |
| 3 | 785 | 135 | 24,4 | 5,6 | 18,2 | 81,3 | 14,8 |
| 4 | 785 | 124 | 30,5 | 8,6 | 24,0 | 78,0 | 18,7 |
| 5 | 786 | 112 | 35,8 | 12,7 | 30,1 | 73,8 | 22,1 |

**Patentansprüche**

1. Verfahren zur kontinuierlichen Herstellung von Cyclohexen durch partielle Hydrierung von Benzol mit Wasserstoff in Gegenwart von Wasser und Rutheniumkatalysatoren bei erhöhter Temperatur und erhöhtem Druck, dadurch gekennzeichnet, daß man Benzol gasförmig zuführt und gebildetes Cyclohexen gasförmig austrägt, wobei der Katalysator in einer flüssigen, wäßrigen Phase gelöst oder suspendiert vorliegt.

2. Verfahren nach Anspruch 1, dadurch gekennzeichnet, daß man die Umsetzung bei einer Temperatur im Bereich von 20 bis 300°C durchführt.

3. Verfahren nach Anspruch 1 oder 2, dadurch gekennzeichnet, daß man die Umsetzung bei einem Gesamtdruck im Bereich von 0,1 bis 20 MPa durchführt.

4. Verfahren nach einem der Ansprüche 1 bis 3, dadurch gekennzeichnet, daß man die Umsetzung bei einem Wasserstoffpartialdruck im Bereich von 50 kPa bis 5 MPa durchführt.

5. Verfahren nach einem der Ansprüche 1 bis 4, dadurch gekennzeichnet, daß man die Umsetzung bei einem Benzolpartialdruck im Bereich von 10 kPa bis 1 MPa durchführt.

6. Verfahren nach einem der Ansprüche 1 bis 5, dadurch gekennzeichnet, daß man die Umsetzung bei einem Gewichtsverhältnis von Wasser zu Katalysator im Bereich von 5:1 bis 1000:1 durchführt.

**Claims**

1. A process for the continuous preparation of cyclohexene by partial hydrogenation of benzene with hydrogen in the presence of water and a ruthenium catalyst at elevated temperatures and superatmospheric pressure, wherein benzene is introduced in gaseous form and the resulting cyclohexene is discharged in gaseous form, the catalyst being present in solution or suspension in a liquid, aqueous phase.

2. A process as claimed in claim 1, wherein the reaction is carried out at from 20 to 300°C.

3. A process as claimed in claim 1 or 2, wherein the reaction is carried out at a total pressure of from 0.1 to 20 MPa.

4. A process as claimed in any of claims 1 to 3, wherein the reaction is carried out at a hydrogen partial pressure of from 50 kPa to 5 MPa.

**5.** A process as claimed in any of claims 1 to 4, wherein the reaction is carried out at a benzene partial pressure of from 10 kPa to 1 MPa.

**6.** A process as claimed in any of claims 1 to 5, wherein the reaction is carried out at a weight ratio of water to catalyst of from 5:1 to 1000:1.

**Revendications**

**1.** Procédé de préparation continue de cyclohexène par hydrogénation partielle de benzène avec de l'hydrogène en présence d'eau et de catalyseurs au ruthénium à température élevée et à pression élevée, caractérisé en ce qu'on introduit le benzène sous forme gazeuse et en ce qu'on évacue le cyclohexène formé sous forme gazeuse, le catalyseur se trouvant sous forme solubilisée ou mise en suspension dans une phase aqueuse liquide.

**2.** Procédé selon la revendication 1 caractérisé en ce qu'on effectue la conversion à une température dans la gamme de 20 à 300 °C.

**3.** Procédé selon la revendication 1 ou 2, caractérisé en ce qu'on effectue la conversion à une pression totale dans la gamme de 0,1 à 20 MPa.

**4.** Procédé selon l'une des revendications 1 à 3, caractérisé en ce qu'on effectue la conversion à une pression partielle d'hydrogène dans la gamme de 50 kPa à 5 MPa.

**5.** Procédé selon l'une des revendications 1 à 4, caractérisé en ce qu'on effectue la conversion à une pression partielle de benzène dans la gamme de 10 kPa à 1 MPa.

**6.** Procédé selon l'une des revendications 1 à 5, caractérisé en ce qu'on effectue la conversion à un rapport pondéral d'eau au catalyseur dans la gamme de 5 : 1 à 1.000 : 1.

Fig. 1

☐   Beispiele 1a bis 1c (Kontinuierlicher Rührkessel; differentielle Selektivität)

...   Beispiele 1a bis 1c (integrale Selektivität)

○   Beispiele 42 bis 44 aus der US 4,678,861, (integrale Selektivität)

△   Beispiele 3a bis 3e (Rührkesselkaskade)